# EUROPEAN PATENT SPECIFICATION

(11) **EP 4 258 987 B1**
(45) Date of publication and mention of the grant of the patent: **29.01.2025**
(21) Application number: 21827667.3
(22) Date of filing: 13.12.2021
(51) Int. Cl.: A61B 5/08, A61B 5/00

(54) **INTRA ORAL RESPIROMETER**
INTRAORALES RESPIROMETER
RESPIROMÈTRE INTRA-ORAL

(30) Priority: 14.12.2020 US 202063125112 P
(43) Date of publication of application: 18.10.2023
(73) Proprietor: Politecnico di Milano, 20133 Milano (IT)
(72) Inventor: ALIVERTI, Andrea, 22100 Como (CO) (IT); SORESINI, Giulia, 20139 Milano (MI) (IT); ESMAILBEIGI, Hananeh, Chicago, Illinois 60654 (US)
(74) Representative: Gatti, Enrico
(86) International application number: PCT/IB2021/061625
(87) International publication number: WO 2022/130164

(56) References cited:
- WO-A1-2020/113345
- US-A1- 2019 175 104
- US-A1- 2020 093 571
- NABAVI SEYEDFAKHREDDIN ET AL: "Oral Cavity Pressure Measurement-based Respiratory Monitoring System with Reduced Susceptibility to Motion Artifacts", 2020 42ND ANNUAL INTERNATIONAL CONFERENCE OF THE IEEE ENGINEERING IN MEDICINE & BIOLOGY SOCIETY (EMBC), IEEE, 20 July 2020 (2020-07-20), pages 5900 - 5904, XP033816362, DOI: 10.1109/EMBC44109.2020.9176425

## Description

The present invention refers to an intra oral respirometer and more in particular to a wearable ventilatory monitoring system.

The human respiratory system is unique. It serves basic life-support functionalities, working in conjunction with the circulatory system. It supplies the body with the oxygen needed and disposes of waste substances. Ventilatory parameters are thus fundamental to monitor, to determine the overall health status of an individual. Ventilatory parameters include the respiratory rate (RR), which is one of the four vital signs, and breath-by-breath amplitude, or tidal volume, but also other derived parameters such as the minute-by-minute respiratory rate.

A wearable and non-invasive monitoring system for ventilatory parameters is thus imperative, to continuously track potentially dangerous variations in vital signs.

Various technologies are already available on the market for assessing such parameters. These include, but are not limited to, spirometry, thermal methods, acoustic or gas-based methods and thoracic motion analysis.

However, all these methods are either too invasive, requiring the use of a mouthpiece connected to an external machine, that could hinder the physiological movements, or perform totally indirect measures often affected by motion artefacts, mainly indoors, in a clinical setting or a laboratory.

In addition, most of them are only limited to the monitoring of one parameter, the respiratory rate, overlooking other important information about the subject respiratory system status.

The object of the present invention is to provide an intra oral respirometer and more in particular a wearable ventilatory monitoring system which is able to overcome the drawback if the prior art.

The article of NABAVI SEYEDFAKHREDDIN ET AL: "Oral Cavity Pressure Measurement-based Respiratory Monitoring System with Reduced Susceptibility to Motion Artifacts", 2020 42ND ANNUAL INTERNATIONAL CONFERENCE OF THE IEEE ENGINEERING IN MEDICINE & BIOLOGY SOCIETY (EMBC), IEEE, 20 July 2020 (2020-07-20), pages 5900-5904, discloses a pressure sensor based noninvasive intraoral wearable system for human respiratory monitoring. The system offers a way of respiratory monitoring by measuring the pressure of the oral cavity. The system consists of a sensing unit which measures the oral cavity pressure and is placed between the inner sides of the upper jaw such that the top surface of the pressure sensor (sensing unit) faces the upper hard palate and there is an air gap between the upper palate and the pressure sensor and a reader and data transmission unit comprising a Bluetooth LE module. The documents WO2020/113345, US2019/175104, US2020/093571 disclose similar devices.

In accordance with the present invention, said objects and others are achieved by a wearable ventilatory monitoring system according to claim 1.

Said objects are furthermore achieved by a method for measuring the respiratory rate of a patient by means of a wearable ventilatory monitoring system according to claim 11.

Further characteristics of the invention are described in the dependent claims.

This solution proposes an innovative Bluetooth-enabled, oral wearable ventilatory monitoring system. The proposed solution is very portable and discreet, suitable for continuous monitoring of both respiratory rate, breath-by-breath amplitude and other derived parameters, even outside the clinical context, and during various daily-life activities, including sleeping. The Intra-Oral Respirometer directly measures the pressure variations during the breathing cycle, with no need for complex algorithms to recover the breathing pattern, for example, from movements of the thoracic area.

The characteristics and advantages of the present invention will be evident from the following detailed disclosure of a practical embodiment thereof, illustrated by way of non-limiting example in the attached drawings, in which:

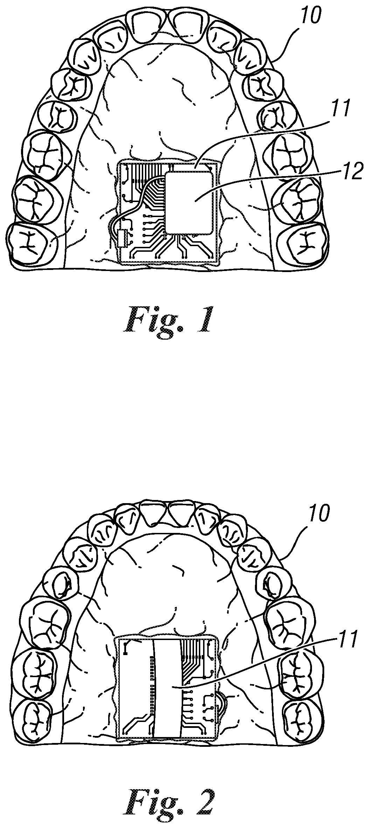

figure 1 shows a wearable ventilatory monitoring system, top view, in accordance with the present invention;
figure 2 shows a wearable ventilatory monitoring system, bottom view, in accordance with the present invention;

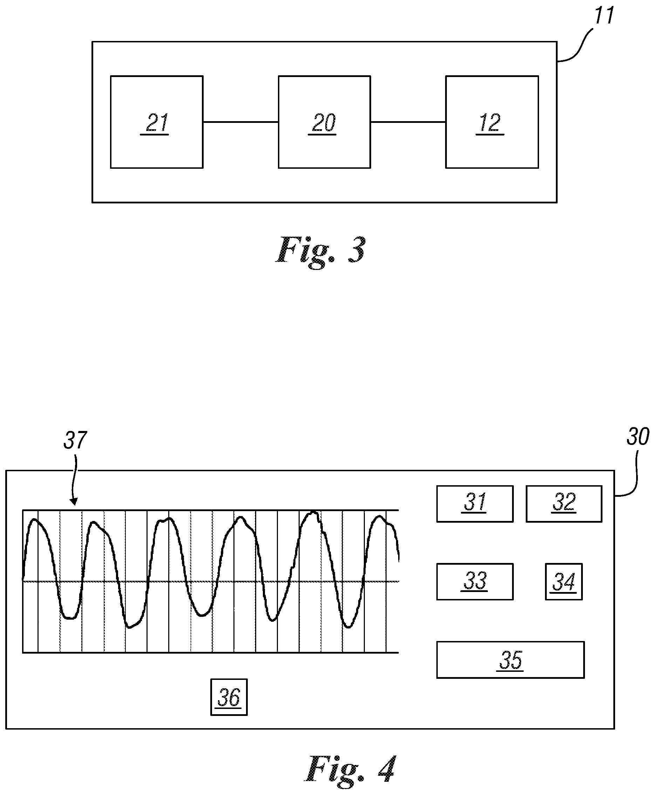

figure 3 shows a block diagram of a wearable ventilatory monitoring system, in accordance with the present invention;
figure 4 shows a possible printout of the data obtained from a wearable ventilatory monitoring system, in accordance with the present invention.

With reference to the accompanying figures, a wearable ventilatory monitoring system, in accordance with the present invention, comprises a flexible dental prosthesis 10 on whose palate is secured an electronic circuit 11.

The electronic circuit 11 comprises a Low Energy (BLE) 20 circuit and a pressure sensor 21, with a battery 12.

The BLE 20, comprises a chip, passive components, and an on-board crystal oscillator. A 32 GPIO (General Purpose Input Output) are provided, and an 8 ADC (Analog-to-Digital Converter) pins to connect sensors and components. The BLE technology is a Bluetooth that works at the same frequency, 2.4 GHz, of the common Bluetooth, being at the same time cheaper to use and implement and consuming less power. The module ensures a UART (Universal Asynchronous Receiver/Transmitter) serial connection over BLE, acting as the peripheral. A computer or a mobile phone acts as the central device of control receiving all the data and elaborating a showing it.

The pressure sensor 21 is preferably a miniature piezo-resistive pressure that it is able to measure also the temperature, and therefore provides a high-resolution pressure and temperature reading. The pressure sensor 21 is fabricated to be resistant to water, as it is protected by a gel and stainless antimagnetic steel cap, allowing a direct exposure to saliva without harming the subject nor affecting the readings. The module is 6.4 mm x 6.2 mm, with a thickness of 2.88 mm, thus greatly meeting the spatial requirements for this project, without excessively hindering the physiological movements of the tongue within the oral cavity. The sensor provides a 24-bit pressure and temperature value, through an ultra-low power ΔΣ ADC.

The pressure sensor 21 provides high resolution from 0.45 to 0.15 mbar, considering that during spontaneous breathing of about 0.5 L, the alveolar pressure varies between +/- 1 mmHg. In addition, the conversion time is as fast as 1 ms and the power required is low (1µA when working, 0.15 µA in standby). Considering that when advertising the BLE module consumes 80 µA, and when connected via BLE 900 µA.

The pressure sensor 21 measures the temperature too. Besides being used to compensate the pressure value, the temperature reading can be quite useful to study the correlation between the two signals. During the breathing cycle, the inhaled air is normally cooler than the exhaled air. This characteristic can be fundamental to perform sensor fusion and enhance the information gained from the device.

The power battery 12, which comprise preferably of a regulator, is a battery of 3.7 V (70 mAh) LiPo.

The Regulator Circuit is used to ensure the sensor receives a voltage of 3.3 V all the time. The LiPo (Lithium ion Polymer) battery used to power the board provides 3.7 V

With the expected consumption of the circuits, the LiPo battery 12 is expected to last for a total of 13 hours during full use of the device.

The final product required a custom-made board to minimize the overall footprint of the device. SMD, Surface Mount Device, components were arranged on the board to minimize the overall footprint. The board was assembled using a hot air reflow soldering station. After soldering, excess solder and solder bridges were removed. Both the board and the solder paste used were lead free, to minimize the hazard for the subjects.

To ensure that the electronic circuit 11 is hermetically insulated from the oral cavity environment, it is entirely embedded in a plastic sheet, attached to a subject-specific dental prosthesis 10 using dental tape, to guarantee perfect adherence and avoid casual detachments. The electronic circuit 11 was positioned toward the posterior end of the plastic dental prosthesis 10. Thus, once inserted in the oral cavity, the site of measure is at the end of the hard palate, allowing the device to perform readings both during oral and nasal breathing.

The dental prosthesis 10 was obtained from the impression of the subject's upper palate and front teeth line.

In particular, a Vinyl Polysiloxane Impression Putty was used to obtain an impression of the subject's upper palate and front teeth line. The subject was instructed to bite down on the tray, placed on the upper palate, for three minutes. The tray was removed and allowed to set for 15 minutes at room temperature. A urethane resin was mixed thoroughly and poured in the dental mold, coated with a mold release spray. After two hours the mold was removed and refined with a file and added clay. Once the cast was ready, the dental prosthesis 10 was produced with a vacuum forming machine using a single sheet of plastic biocompatible. Hot air was used to properly seal any gaps or defects and the retainer was removed from the cast. After cutting the remaining edges, and filing excess parts, the retainer was sanitized with alcohol.

The system can also host a variety of other sensors, and in particular a sensor to monitor O2 and CO2 concentration. These would allow, together with the flow measurement, estimated by assuming a linear relationship with the pressure values, to determine oxygen consumption and carbon dioxide production on a breath-by-breath basis. Oxygen consumption can be computed as the difference between the oxygen volume that enters lung during inhalation and the oxygen volume that exits during exhalation. In addition, a humidity sensor could be included in the device. Adding such sensors would allow to perform sensor fusion using different types of measures, thus enhancing and improving the information gained from the device.

The system require a software for the working of the BLE 20 and a software to provide the subjects with a user-friendly visualization tool.

The software for the working of the BLE 20 comprises two portions. When the device first connects, after initialization of all constants and user-controlled variables, a custom calibration loop is run for 10 seconds. Sensor's readings are sampled at 12 Hz, which greatly meets the requirements of the Nyquist criterion. The spontaneous breathing rate is around 1 Hz; thus, the sampling frequency is 12 times the maximum signal frequency when a subject is breathing normally. Considering that Nyquist requires a theoretical sampling frequency of at least two times the maximum frequency of the signal, and an empirical one of five times, a sampling frequency of 12 Hz is believed to fulfil the requirements even for faster breathing patterns. The pressure values are stored in an array for the duration of the entire calibration phase. After 10 seconds, the average pressure value is computed. The reading phase starts right after calibration ends. The average pressure computed is subtracted to each pressure reading to only account for pressure variations caused by inspiration and expiration, eliminating the atmospheric pressure value. The atmospheric pressure at sea level is around 1013.25 mbar or cmH2O, thus it would mask a pressure oscillation of around 1/2 mbar during the breathing cycle. Both pressure and temperature data are stored as JSON, JavaScript Object Notation, a light open standard data-interchange format, easy to write and read for humans, and especially easy to parse for machines.

The use of the JSON allows data to be easily parsed and appropriately displayed when the Web Application is started.

To assess the usability of the system and to provide users with a user-friendly interface 30, a Web App was developed using Web Bluetooth, HTML and JavaScript. Given the tools used to develop the application, it can be used both on a computer screen and on a mobile phone. The only requirement being the installation of Chrome. This way the live monitoring can be performed both at home and outside, allowing the user to utilize the proposed device throughout all daily activities.

Typically the interface 30 comprises a button 31 for connect the interface to the device and a button 32 for disconnecting it. The age must be inserted in the box 33 and push the button 34.

The interface will show in the box 35 the temperature, and in the box 36 the respiratory rate and in addition will show the live plot 37 of the respiratory signal.

The Web Application can directly connect to the board over BLE, using the Web Bluetooth technology. The BLE chip acts as the GATT (Generic Attribute Profile) server, as it provides the services. The laptop acts the client, that connects to the GATT server. The HTML code includes a JavaScript file, that contains the Bluetooth characteristics, of the BLE chip. When clicking on the "Connect" button, the "reading" function is called. The function first connects the Web App, the client, to the device, or GATT server. Once connection is established, the JSON received is parsed.

The temperature value read from the sensor is then displayed in a gauge, without any pre-processing step. The pressure signal, on the other hand, needs some signal processing steps before being plot.

It is known that the respiratory signal resembles a sinusoid, with peaks corresponding to the end of inspiration (minimum) and the end of expiration (maximum), when measuring pressure. A FIR, Finite Impulse Response, filter was designed to eliminate noise and obtain a sinusoid. This type of filter guarantees a finite impulse response and no phase distortion, differently from the IIR filter. A low-pass filter of order 11 (to limit the delay) was used to remove the high frequency noise affecting the signal, with a cut-off frequency of 0.25 Hz.

Considering the average frequency of the respiratory signal during spontaneous breathing, the filter was strong enough to attenuate noise. At 1 Hz the signal is attenuated of around 10 dB, meaning that a slight decrease in signal amplitude is expected. However, this did not affect our first aim of monitoring respiratory rate.

Given the necessity of providing a live monitoring of the respiratory frequency, the strategy adopted was the implementation of an algorithm to count the number of maxima (expiration) and minima (inspiration) in 10 seconds of signal. The respiratory rate was approximated as the average of these counts multiplied by 6, to obtain the number of breaths in a minute. Both maxima and minima were considered to provide a more accurate count.

This way the user can be immediately informed whether something is wrong. A message appears on the screen both when hyperpnoea and hypopnea happen. However, to correct potential errors, rising from missing maxima or minima, or accounting for the same maximum or minimum twice, the respiratory rate displayed is adjusted every minute to the correct number.

In addition, the swallowing problem was addressed. On average, an adult swallows 1-2 times per minute, touching the palate with the tongue. This produces a spike in both the pressure and temperature signal, which needs to be accounted for. Given the noticeable difference between a real inspiration or expiration and a swallowing artefact, the algorithm developed simply removes values higher than a pre-determined threshold (+/- 6 cmH2O), when computing the respiratory rate. Considering that the pharynx closes the trachea during swallowing, thus temporarily pausing breathing, our approach does not eliminate any useful information.

The users were also asked to insert their age, to provide the appropriate range for respiratory range, as in Table I.

Children below 6 years were not included in the age range, as the device is believed to be unsafe for small children to wear, as they could swallow it unwillingly. The Table I was used to compute the appropriate thresholds to determine the potential presence of abnormal respiratory rates.

The temperature value was acquired to study the correlation with the pressure signal. In addition, it represents an accurate indicator of the general health status of the subject. Fever is, indeed, the first indicator of an infection or other disease. Since the sensor is placed in close contact with the subject's oral mucosa, the temperature value is normal between 35.5 °C and 37.5 °C.

The measures of this system were validated by comparison with an Optoelectronic Plethysmography (OEP).

Various conditions and modalities were accounted for and environmental effects were considered.

The comparison suggest that this system performs accurate measurements in most conditions, and with some degree of inaccuracy is still able to detect changes in ventilation, caused by variations in the tidal volume, in different contexts.

The developed device was also capable of monitoring ventilatory parameters both while sitting and while exercising, proving its capability for use outside the clinical context. Particularly, the ability of performing measurements during a physical effort, even without the use of an external machine, and without being affected by motion artefacts, constitutes a great advantage.

The device was demonstrated not only to be able of monitoring respiratory rate in various conditions, including extreme exercise, but also of providing accurate values of breath.

| TABLE I | |
|---|---|
| Age related ranges for respiratory range. [years] | Normal Respiratory Rate [bpm] |
| 6-9 | 18-25 |
| 10-17 | 17-23 |
| 18-64 | 12-20 |
| 65-79 | 12-28 |
| >80 | 10-30 |

## Claims

1. A wearable ventilatory monitoring system comprising:
an electronic circuit (11);
said electronic circuit (11) comprises
a Bluetooth Low Energy (20) circuit,
a pressure sensor (21), and
a battery (12);
wherein
said electronic circuit (11) is secured on a flexible plastic dental prosthesis (10) toward the posterior end of said plastic dental prosthesis (10);
said electronic circuit (11) is entirely embedded in a plastic sheet;
said pressure sensor (21) is configured to measure pressure variation during the breathing cycle;
said pressure sensor (21) is protected by a gel and stainless antimagnetic steel cap.

2. The system according to claim 1 **characterised in that** said pressure sensor (21) is of the type piezo-resistive.

3. The system according to claim 1 **characterised in that** said electronic circuit (11) comprises temperature sensor.

4. The system according to one of the preceding claims **characterised in that** said Bluetooth Low Energy (20) circuit is able to communicate with a computer or a mobile phone that acts as a central device of control receiving all the data measured by said pressure sensor (21).

5. The system according to claim 4 **characterised in that** said a computer or a mobile phone calculate the respiratory rate of a patient by the measure received from said pressure sensor (21).

6. The system according to claim 5 **characterised in that** said a computer or a mobile phone calculate the respiratory rate of a patient removing values, of the measure received from said pressure sensor (21), higher than a pre-determined threshold.

7. The system according to claim 1 **characterised in that** said flexible dental prosthesis (10) is done with a biocompatible plastic.

8. The system according to claim 1 **characterised in that** said battery (12) is rechargeable.

9. The system according to claim 1 **characterised in that** said electronic circuit (11) is hermetically insulated from the oral cavity environment.

10. The system according to claim 1 **characterised in that** said electronic circuit (11) is attached said flexible dental prosthesis (10) using dental tape.

11. A method for measuring the respiratory rate of a patient comprising a step of measuring the respiratory rate of a patient by means of a wearable ventilatory monitoring system according to claim 1.

## Patentansprüche

1. Ein tragbares Beatmungsüberwachungssystem, welches Folgendes umfasst:
eine Elektronikschaltung (11);
wobei die genannte Elektronikschaltung (11) jeweils Folgendes umfasst
eine Bluetooth Niedrigenergieschaltung (20),
einen Drucksensor (21) und
eine Batterie (12);
wobei die genannte Elektronikschaltung (11) an einer flexiblen Kunststoff-Zahnprothese (10) in Richtung des hinteren Endes der genannten Kunststoff-Zahnprothese (10) befestigt ist;
die genannte Elektronikschaltung (11) vollkommen in einer Kunststofffolie eingeschlossen ist;
der genannte Drucksensor (21) konfiguriert ist, um die Druckänderung während des Atmungsablaufs zu messen;
der genannte Drucksensor (21) durch ein Gel und eine rostfreie antimagnetische Kappe aus Stahl geschützt wird.

2. Das System gemäß Anspruch 1, **dadurch gekennzeichnet, dass** der genannte Drucksensor (21) als piezoresistiver Typ ausgeführt ist.

3. Das System gemäß Anspruch 1, **dadurch gekennzeichnet, dass** die genannte Elektronikschaltung (11) einen Temperatursensor umfasst.

4. Das System gemäß einem der vorausgegangenen Ansprüche, **dadurch gekennzeichnet, dass** die genannte Bluetooth-Niedrigenergieschaltung (20) in der Lage ist, mit einem Computer oder einem Mobilphon zu kommunizieren, welche als zentrale Steuervorrichtung fungieren und sämtliche durch den genannten Drucksensor (21) gemessenen Daten empfangen.

5. Das System gemäß Anspruch 4, **dadurch gekennzeichnet, dass** der genannte Computer oder das Mobilphon die Atemfrequenz eines Patienten anhand der von dem genannten Drucksensor (21) empfangenen Messung berechnen.

6. Das System gemäß Anspruch 5, **dadurch gekennzeichnet, dass** der genannte Computer oder das Mobilphon die Atemfrequenz eines Patienten durch Abziehen der Werte der von dem genannten Drucksensor (21) empfangenen Messung, welche jeweils höher als eine vorbestimmte Schwelle sind, berechnen.

7. Das System gemäß Anspruch 1, **dadurch gekennzeichnet, dass** die genannte flexible Zahnprothese (10) aus biokompatiblem Kunststoff besteht.

8. Das System gemäß Anspruch 1, **dadurch gekennzeichnet, dass** die genannte Batterie (12) aufladbar ist.

9. Das System gemäß Anspruch 1, **dadurch gekennzeichnet, dass** die genannte Elektronikschaltung (11) hermetisch von dem Umfeld der Mundhöhle isoliert ist.

10. Das System gemäß Anspruch 1, **dadurch gekennzeichnet, dass** die genannte Elektronikschaltung (11) an der genannten flexiblen Zahnprothese (10) mit Hilfe von Zahnklebeband befestigt ist.

11. Ein Verfahren für die Messung der Atemfrequenz eines Patienten, welches den Schritt der Messung der Atemfrequenz eines Patienten mit Hilfe eines tragbaren Beatmungsüberwachungssystems gemäß Anspruch 1 umfasst.

## Revendications

1. Système de surveillance ventilatoire portable comprenant:
un circuit électronique (11);
ledit circuit électronique (11) comprend
un circuit à faible énergie Bluetooth (20),
un capteur de pression (21), et
une batterie (12);
dans lequel
ledit circuit électronique (11) est sécurisé sur une prothèse dentaire en plastique flexible (10) vers l'extrémité arrière de ladite prothèse dentaire en plastique (10);
ledit circuit électronique (11) est entièrement intégré dans une feuille en plastique;
ledit capteur de pression (21) est configuré pour mesurer la variation de pression pendant le cycle respiratoire;
ledit capteur de pression (21) est protégé par un gel et un bouchon en acier inoxydable amagnétique.

2. Système selon la revendication 1, **caractérisé en ce que** ledit capteur de pression (21) est du type piézorésistif.

3. Système selon la revendication 1, **caractérisé en ce que** ledit circuit électronique (11) comprend un capteur de température.

4. Système selon l'une des revendications précédentes, **caractérisé en ce que** ledit circuit à faible énergie Bluetooth (20) est capable de communiquer avec un ordinateur ou un téléphone mobile qui agit comme un dispositif central de commande recevant toutes les données mesurées par ledit capteur de pression (21).

5. Système selon la revendication 4, **caractérisé en ce que** lesdits un ordinateur ou un téléphone mobile calculent la fréquence respiratoire d'un patient par le biais de la mesure reçue par ledit capteur de pression (21).

6. Système selon la revendication 5, **caractérisé en ce que** lesdits un ordinateur ou un téléphone mobile calculent la fréquence respiratoire d'un patient en supprimant des valeurs, de la mesure reçue par ledit capteur de pression (21), supérieures à un seuil prédéterminé.

7. Système selon la revendication 1, **caractérisé en ce que** ladite prothèse dentaire flexible (10) est réalisée avec une matière plastique biocompatible.

8. Système selon la revendication 1, **caractérisé en ce que** ladite batterie (12) est rechargeable.

9. Système selon la revendication 1, **caractérisé en ce que** ledit circuit électronique (11) est isolé hermétiquement de l'environnement de la cavité orale.

10. Système selon la revendication 1, **caractérisé en ce que** ledit circuit électronique (11) est fixé à ladite prothèse dentaire flexible (10) à l'aide d'un ruban dentaire.

11. Procédé de mesure de la fréquence respiratoire d'un patient comprenant une étape de mesure de la fréquence respiratoire d'un patient au moyen d'un système de surveillance ventilatoire portable selon la revendication 1.
